# EUROPEAN PATENT APPLICATION

(11) **EP 4 353 325 A1**
(43) Date of publication of application: **17.04.2024**
(21) Application number: 22820066.3
(22) Date of filing: 26.05.2022
(51) Int. Cl.: A61P 43/00, A61P 17/16, A61Q 19/08, A61K 9/107, A61K 8/06, A61K 8/27, A61K 8/29, A61K 8/49, A61K 8/86, A61K 47/34, A61K 33/06, A61K 33/30, A61K 31/4025

(54) **SKIN CARE COMPOSITION CONTAINING ULTRAVIOLET WAVELENGTH-CONVERTING SUBSTANCE AND ALKYLENE OXIDE DERIVATIVE**

(30) Priority: 09.06.2021 JP 2021096560
(71) Applicant: Shiseido Company, Ltd., Chuo-ku Tokyo 104-0061 (JP)
(72) Inventor: IWAMI, Shiho, Tokyo 104-0061 (JP)
(74) Representative: Cabinet Beau de Loménie
(86) International application number: PCT/JP2022/021651
(87) International publication number: WO 2022/259884

(57) **Abstract**

To provide a novel external skin composition suitable for cell activation.

A external skin composition of the present disclosure contains a UV wavelength conversion substance and an alkylene oxide derivative.

## Description

### FIELD

The present invention relates to an external skin composition having a cell activation action and containing a UV wavelength conversion substance and an alkylene oxide derivative, and a UV wavelength conversion function promoter of a UV wavelength conversion substance, which contains an alkylene oxide derivative as an active ingredient.

### BACKGROUND

Ultraviolet is considered to generate free radicals *in vivo* and thereby cause oxidation of sebum and damage to cellular DNA. Examples of the damage caused by ultraviolet to skin include adverse effects such as skin cancer, photoaging, spots, wrinkles, and inflammation. These are undesirable from a health and beauty perspective.

In recent years, it has been discovered that UV wavelength conversion substances not only reduce the harmful effects of ultraviolet rays but also have cell activating effects. For example, emulsion compositions containing UV wavelength conversion substances have been reported to activate cells by increasing the concentration of cytochrome c in human skin-derived fibroblasts and increasing mitochondrial activity when exposed to sunlight including ultraviolet rays (Patent Literature 1).

Alkylene oxide derivatives are known as non-sticky moisturizers and are used in external skin preparations (Patent Literature 2).

### [CITATION LIST]

### [PATENT LITERATURE]

[PTL 1] WO 2020/204196
[PTL 2] Japanese Unexamined Patent Publication (Kokai) No. 2004-083541

### SUMMARY

### [TECHNICAL PROBLEM]

An object of the present invention is to provide a novel external skin composition having a cell activation action using ultraviolet rays, and provide a UV wavelength conversion function promoter of a UV wavelength conversion substance using an alkylene oxide derivative as an active ingredient.

### [SOLUTION TO PROBLEM]

The present inventors have conducted extensive research in order to utilize ultraviolet rays effectively for the skin. As a result, the present inventors have discovered that alkylene oxide derivatives promote the function of a UV wavelength conversion substance, and conceived of a novel external skin composition having excellent cell activation action and a UV wavelength conversion function promoter of a UV wavelength conversion substance, which contains an alkylene oxide derivative as an active ingredient.

The present disclosure provides the following inventions.
[1] An external skin composition, comprising:
   (A) a UV wavelength conversion substance, and
   (B) an alkylene oxide derivative represented by formula (I) below:

      R¹O-[(EO)ₘ(AO)ₙ]-R² (I)

      where AO is an oxyalkylene group having 3 to 4 carbon atoms,
      EO is an oxyethylene group,
      the oxyalkylene group having 3 to 4 carbon atoms and the oxyethylene group may be added in a block or random manner,
      m and n are average numbers of added moles of the oxyethylene group and the oxyalkylene group, respectively, and 1 ≤ m ≤ 70, 1 ≤ n ≤ 70, and 0.2 ≤ m/(m + n) ≤ 0.8,
      R¹ and R² are an alkyl group having 1 to 4 carbon atoms or a hydrogen atom, and may be the same or different,
      the ratio of the number of hydrogen atoms in R¹ and R² to the number of alkyl groups in R¹ and R² is 0.15 or less on average.
[2] The external skin composition according to [1], wherein the UV wavelength conversion substance is one or more selected from a zinc oxide phosphor, a magnesium titanate phosphor, and phycocyanin.
[3] The external skin composition according to [1] or [2], wherein the UV wavelength conversion substance is a zinc oxide phosphor and phycocyanin.
[4] The external skin composition according to any one of [1] to [3], wherein the oxyalkylene group and the oxyethylene group of the alkylene oxide derivative are added in a random manner.
[5] The external skin composition according to any one of [1] to [4], wherein the oxyalkylene group of the alkylene oxide derivative is an oxypropylene group.
[6] The external skin composition according to any one of [1] to [5], wherein R¹ and R² of the alkylene oxide derivative are both methyl groups.
[7] The external skin composition according to any one of [1] to [6], comprising 1% by weight or more of the alkylene oxide derivative.
[8] The external skin composition according to any one of [1] to [7], which is a water-in-oil composition.
[9] The external skin composition according to any one of [1] to [8], which is for cell activation.
[10] A UV wavelength conversion function promoter of a UV wavelength conversion substance, comprising an alkylene oxide derivative represented by the following general formula (I) as an active ingredient:

   R¹O-[(EO)ₘ(AO)ₙ]-R² (I)

   where AO is an oxyalkylene group having 3 to 4 carbon atoms,
   EO is an oxyethylene group,
   the oxyalkylene group having 3 to 4 carbon atoms and the oxyethylene group may be added in a block or random manner,
   m and n are average numbers of added moles of the oxyethylene group and the oxyalkylene group, respectively, and 1 ≤ m ≤ 70, 1 ≤ n ≤ 70, and 0.2 ≤ m/(m + n) ≤ 0.8,
   R¹ and R² are an alkyl group having 1 to 4 carbon atoms or a hydrogen atom, and may be the same or different,
   the ratio of the number of hydrogen atoms in R¹ and R² to the number of alkyl groups in R¹ and R² is 0.15 or less on average.
[11] The UV wavelength conversion function promoter according to [10], wherein the UV wavelength conversion substance is one or more selected from a zinc oxide phosphor, a magnesium titanate phosphor, and phycocyanin.
[12] The UV wavelength conversion function promoter according to [10] or [11], wherein the oxyalkylene group and the oxyethylene group of the alkylene oxide derivative are added in a random manner.
[13] The UV wavelength conversion function promoter according to any one of [10] to [12], wherein the oxyalkylene group of the alkylene oxide derivative is an oxypropylene group.
[14] The UV wavelength conversion function promoter according to any one of [10] to [13], wherein R¹ and R² of the alkylene oxide derivative are both methyl groups.
[15] Use of an alkylene oxide derivative represented by the following formula (I), for promoting a function of a UV wavelength conversion substance:

   R¹O-[(EO)ₘ(AO)ₙ]-R² (I)

   where AO is an oxyalkylene group having 3 to 4 carbon atoms,
   EO is an oxyethylene group,
   the oxyalkylene group having 3 to 4 carbon atoms and the oxyethylene group may be added in a block or random manner
   m and n are average numbers of added moles of the oxyethylene group and the oxyalkylene group, respectively, and 1 ≤ m ≤ 70, 1 ≤ n ≤ 70, and 0.2 ≤ m/(m + n) ≤ 0.8,
   R¹ and R² are an alkyl group having 1 to 4 carbon atoms or a hydrogen atom, and may be the same or different,
   the ratio of the number of hydrogen atoms in R¹ and R² to the number of alkyl groups in R¹ and R² is 0.15 or less on average.

### [ADVANTAGEOUS EFFECTS OF INVENTION]

The alkylene oxide derivative, which is a UV wavelength conversion function promoter according to the present invention, is suitable for a UV wavelength conversion substance to convert ultraviolet rays into visible light, whereby strong emitted light (fluorescence) can be obtained. The external skin composition containing an alkylene oxide derivative and a UV wavelength conversion substance of the present invention emits strong fluorescence when irradiated with ultraviolet rays, and can activate skin cells by effectively utilizing ultraviolet rays.

### DESCRIPTION OF EMBODIMENTS

The present invention will be described below in detail with reference to specific embodiments. However, the present invention is not limited to the following embodiments, and can be carried out in any embodiments without departing from the spirit of the present invention.

All of the patent publications, unexamined patent publications, and non-patent literature cited in the present disclosure are incorporated by reference in their entirety into the present disclosure for all purposes.

In the present disclosure, "to" when applied to numerical values refers to a range of values that fall within a range that is greater than or equal to a defined reference value and less than or equal to a defined reference value.

### (A) UV Wavelength Conversion Substance

The external skin composition of the present invention contains a UV wavelength conversion substance as an active ingredient. The phrase "UV wavelength conversion substance" refers to a substance which converts the wavelength of ultraviolet contained in incident light to outgoing light with a wavelength longer than the wavelength of ultraviolet. The phrase "organic UV wavelength conversion substance" refers to a UV wavelength conversion substance which is an organic compound, and the phrase "inorganic UV wavelength conversion substance" refers to a UV wavelength conversion substance which is an inorganic compound.

The ultraviolet may contain UVA, UVB, or UVC. In one embodiment, the ultraviolet is light having a peak wavelength of 200 nm to 400 nm. Further, incident light, for example, sunlight, may contain ultraviolet. Furthermore, the incident light may be ultraviolet or artificially generated ultraviolet may be used.

The outgoing light emitted from the UV wavelength conversion substance has a longer wavelength than ultraviolet and has a peak wavelength of preferably 450 nm to 700 nm or 500 nm to 700 nm. The outgoing light may have one or more peaks, for example, but not limited to, at 510 nm, 520 nm, 530 nm, 540 nm, 550 nm, 560 nm, 570 nm, 580 nm, 590 nm, 600 nm, 610 nm, 620 nm, 630 nm, 640 nm, 650 nm, 660 nm, 670 nm, 680 nm, 690 nm, 700 nm, or within any range therebetween or may be red light, orange light, green light, or blue light. In one embodiment, the main wavelength exhibited by the light emitted from the UV wavelength conversion substance when excited by 200 nm to 400 nm excitation light is 500 nm to 700 nm.

Examples of the UV wavelength conversion substance include the following components: phycobiliproteins such as phycocyanin (allophycocyanin, C-phycocyanin, R-phycocyanin), phycoerythrocyanin, phycoerythrin (B-phycoerythrin, b-phycoerythrin, C-phycoerythrin, R-phycoerythrin); natural or synthetic components such as vitamin A, β-carotene, vitamin K, vitamin B1, vitamin B2, vitamin B6, vitamin B12, folic acid, niacin, salicylic acid, lycopene, capsicum extract, capsicum color, paprika color, gardenia color, safflower color, turmeric color, cochineal color, perilla color, red cabbage color, flavonoid, carotenoid, quinoid, porphyrins, anthocyanins, and polyphenols; colors such as Red No. 401, Red No. 227, Red No. 504, Red No. 218, Orange No. 205P, Yellow No. 4, Yellow No. 5, Green No. 201, pyranine conc., Blue No. 1, 2,4-diaminophenoxyethanol hydrochloride, Alizuline Purple SS, Purple No. 401, Black No. 401, Herindon pink, Yellow No. 401, Benzidine yellow G, Blue No. 404, Red No. 104, and meta-aminophenol; phosphors of an inorganic compound doped to exhibit a fluorescence property, such as the blue phosphor containing an amorphous silica particle, cerium, and phosphorus and/or magnesium described in Japanese Registered Patent Publication No. 6424656, the red phosphor containing a europium-activated compound of a mixed crystal of an alkaline earth metal sulfide and a gallium compound described in Japanese Registered Patent Publication No. 6361416; the zinc oxide phosphor described in WO 2018/004006; the zinc oxide phosphor described in Japanese Unexamined Patent Publication (Kokai) No. 2018-131422; and the inorganic phosphor described in Japanese Unexamined Patent Publication (Kokai) No. 5-117127 (hereinafter, a phosphor derived from zinc oxide is referred to as a "zinc oxide phosphor"). In one embodiment, the inorganic phosphor is one or more phosphors selected from phosphors of zinc oxide represented by ZnO:Zn, Zn_{1+z}, or ZnO₁₋ₓ, doped with a sulfide and/or a sulfate, such as zinc sulfide and zinc sulfate, described in WO 2018/004006; magnesium titanate phosphors of a magnesium titanate such as MgTiO₃ or Mg₂TiO₄ doped with manganese (hereinafter a phosphor derived from magnesium titanate is referred to as a "magnesium titanate phosphor"); and calcium phosphate phosphors of a calcium phosphate such as Ca(H₂PO₄)₂, CaHPO₄, or Ca₃(PO₄)₂ doped with cerium.

Further, the inorganic UV wavelength conversion substance which is an inorganic phosphor may be subjected to a surface treatment. Examples of the surface treatment include a silane compound treatment (octyltriethoxysilane, etc.), a silicone compound treatment, a fluorine-modified silicone compound treatment, a fluorine compound treatment, a higher fatty acid treatment (stearic acid, etc.), a higher alcohol treatment, a fatty acid ester treatment, a metal soap treatment, an amino acid treatment, and an alkyl phosphate treatment.

UV wavelength conversion substance may be obtained by a method of extraction from natural products such as animals, plants, and algae or an artificial method such as chemical synthesis. For example, phycobiliproteins may be prepared from blue-green algae such as Spirulina platensis, or red algae such as Porphyridium cruentum by the extraction method described in, for example, Japanese Registered Patent Publication No. 4048420, Japanese Registered Patent Publication No. 4677250, or Japanese Registered Patent Publication No. 3303942. Zinc oxide phosphors may be produced by the method described in, for example, WO 2018/004006, Japanese Unexamined Patent Publication (Kokai) No. 2018-131422, or Japanese Unexamined Patent Publication (Kokai) No. 5-117127. Magnesium titanate phosphors may be produced by the method described in Japanese Unexamined Patent Publication (Kokai) No. 2017-88719. Calcium phosphate phosphor may be produced by the method described in WO 2018/117117.

These UV wavelength conversion substances may consist of or contain these exemplified components, which may be used alone or in combination of two or more, as long as the wavelength conversion effect of the invention is not impaired. For example, there may be included a phycobiliprotein such as phycocyanin and an inorganic phosphor such as zinc oxide phosphor, and to the phycobiliprotein or inorganic phosphor, there may be added another UV wavelength conversion substance such as vitamin B (vitamin B1, vitamin B2, vitamin B6, or vitamin B12, etc.) to aim for a synergistic effect. Note that these components are merely examples and any substance which can bring about a wavelength conversion effect of the present invention can be used.

Any derivative of vitamin B2, which is a UV wavelength conversion substance, may be used as long as the derivative is a UV wavelength conversion substance. Examples of the vitamin B2 derivatives include riboflavin acetate ester, riboflavin butyrate, riboflavin phosphate (may be a sodium or mono-diethanolamine salt), flavin mononucleotide, flavin adenine dinucleotide, riboflavin tetrabutyrate, and riboflavin tetranicotinate. Derivatives of lixoflavin, which is a stereoisomer of riboflavin, may be used.

The content of the UV wavelength conversion substance in the external skin composition of the present invention is not particularly limited as long as the wavelength conversion effect of the present invention is not impaired. The content can be appropriately determined in accordance with the type of the UV wavelength conversion substance or the application of the external skin composition containing the UV wavelength conversion substance. The range thereof is not limited and may be, for example, 0.001 to 99.99% by weight, 0.001 to 10% by weight, 0.01 to 99.99% by weight, 0.01 to 10% by weight, 0.1% to 99.9% by weight, or 0.1 to 10% by weight.

As one aspect of the present invention, the UV wavelength conversion substance of the external skin composition contains a zinc oxide phosphor. In the external skin composition of the present invention, preferably, the content of a zinc oxide phosphor relative to the total of the external skin composition is 0.1% by weight or more, preferably 1.0% by mass or more, more preferably 1.5% by weight or more, or even more preferably 2% by weight or more, and 20% by weight or less, preferably 15% by weight or less, more preferably 10% by weight or less, or even more preferably 5% by weight or less. The content relative to the total of the external skin composition is 0.01 to 99.99% by weight, 0.1 to 99.9% by weight, 0.1 to 50% by weight, 0.1 to 40% by weight, 0.1 to 30% by weight, 0.1 to 20% by weight, 0.1 to 10% by weight, or 1 to 10% by weight.

As one aspect of the present invention, the UV wavelength conversion substance of the external skin composition contains a magnesium titanate phosphor. In the external skin composition of the present invention, preferably, the content of a magnesium titanate phosphor relative to the total of the external skin composition is 0.1% by weight or more, preferably 1.0% by mass or more, more preferably 1.5% by weight or more, or even more preferably 2% by weight or more, and 20% by weight or less, preferably 15% by weight or less, more preferably 10% by weight or less, or even more preferably 5% by weight or less. The content relative to the total of the external skin composition is 0.01 to 99.99% by weight, 0.1 to 99.9% by weight, 0.1 to 50% by weight, 0.1 to 40% by weight, 0.1 to 30% by weight, 0.1 to 20% by weight, 0.1 to 10% by weight, 0.5 to 10% by weight, or 1 to 10% by weight.

As one aspect of the present invention, the UV wavelength conversion substance of the external skin composition contains phycocyanin. In the external skin composition of the present invention, preferably, the content of a phycocyanin relative to the total of the external skin composition is 0.00001% by weight or more, preferably 0.0001% by mass or more, and 20% by weight or less, preferably 15% by weight or less, more preferably 10% by weight or less, or even more preferably 5% by weight or less. The content relative to the total of the external skin composition is 0.00001 to 99.99% by weight, 0.0001 to 99.9% by weight, 0.0001 to 50% by weight, 0.0001 to 40% by weight, 0.0001 to 30% by weight, 0.0001 to 20% by weight, 0.0001 to 10% by weight, 0.0001 to 5% by weight, 0.001 to 5% by weight, 0.01 to 5% by weight, 0.05 to 5% by weight, 0.1 to 5% by weight, 0.1 to 3% by weight, or 0.1 to 1% by weight.

As one aspect of the present invention, the UV wavelength conversion substance of the external skin composition contains vitamin B2. In the external skin composition of the present invention, preferably, the content of vitamin B2 relative to the total of the external skin composition is 0.00001% by weight or more, preferably 0.0001% by weight or more, and 20% by weight or less, preferably 15% by weight or less, more preferably 10% by weight or less, or even more preferably 5% by weight or less. The content relative to the total of the external skin composition is 0.00001 to 99.99% by weight, 0.0001 to 99.9% by weight, 0.0001 to 50% by weight, 0.0001 to 40% by weight, 0.0001 to 30% by weight, 0.0001 to 20% by weight, 0.0001 to 10% by weight, 0.0001 to 5% by weight, 0.001 to 5% by weight, 0.005 to 5% by weight, 0.01 to 5% by weight, 0.01 to 1% by weight, 0.01 to 0.5% by weight, or 0.01 to 0.1% by weight.

Examples of cell activation include, but are not limited to, promoting metabolism and turnover, improving a function, promoting proliferation, inhibiting oxidation, improving resistance to fatigue and external stimuli, and inhibiting loss of function and activity in cells, such as dermal fibroblasts and/or keratinocytes, of animals including humans. When skin cells are activated, effects such as prevention of or improvement from wrinkles, spots, skin aging, and photoaging can be expected.

The cell activation effect may be performed by measuring, for example, the viability, reducing ability, or proliferation of living cells using AlamarBlue as in Patent Literature 1. Any method can be used such as another dye assay, mitochondrial membrane potential-dependent dye assay, intracellular cytochrome c assay, elastase cleavage dye assay, ATP, ADE assay, glycolytic flux and oxygen consumption assay, collagen assay, photoaging assay, collagen glycation assay, inflammatory substances (interleukin 1α, interleukin 8, tumor necrosis factor α) assay, skin barrier function-related protein (comeodesmosin, sphingomyelin phosphodiesterase, filaggrin, involucrin, loricrin, transglutaminase 1, caspase 14) assay, angiogenesis modulator (VEGF-A, ANGPT1) assay, oxidation and/or skin stress-related protein (aromatic hydrocarbon receptor repressor, cytochrome P4501B1, aromatic hydrocarbon receptor repressor, heme oxygenase 1) assay, or hyaluronic acid assay.

The external skin composition of the present invention is suitable for performing the function of a UV wavelength conversion substance and for alleviating, or more positively improving and recovering, skin damage during and after ultraviolet irradiation by activating cells. Specifically, the external skin composition of the present invention is suitable for collagen production or hyaluronic acid production by fibroblasts and inhibition of damage caused by photoaging and for inhibiting oxidation stress of keratinocytes, enhancing a barrier function, suppressing an inflammatory reaction, and suppressing the glycation of collagen and angiogenesis in skin.

Fluorescence intensity can be measured, for example, using a spectrofluorometer by irradiating with ultraviolet a plate containing the external skin composition, or by forming a coating film of the composition on the surface of a substrate and measuring the fluorescence intensity during irradiation with ultraviolet using a spectrofluorometer. The substrate may be a resin substrate composed of a polymethyl methacrylate (PMMA), nylon, or acrylic plate or an inorganic plate composed of glass or quartz. For example, a PMMA plate having V-shaped grooves on its surface (also refer to as "S plate": Japanese Registered Patent Publication No. 4453995) can be used. As the fluorescence intensity, a fluorescence value at a specific single wavelength or an integrated value in a specific wavelength region may be used.

### (B) Alkylene Oxide Derivative

In the present invention, in particular, it is preferable that among alkylene oxide derivatives, an alkylene oxide derivative represented by the following formula (I) be used:

R¹O-[(EO)ₘ(AO)ₙ]-R² (I)

where AO is an oxyalkylene group having 3 to 4 carbon atoms, EO is an oxyethylene group, m and n are the average numbers of added moles of the oxyethylene group and the oxyalkylene group, respectively, and are 1 ≤ m ≤ 70 and 1 ≤ n ≤ 70; the ratio of oxyethylene groups to the total of oxyalkylene groups having 3 to 4 carbon atoms and oxyethylene groups is 20 to 80% by mass (0.2 ≤ m / (m + n) ≤ 0.8); the oxyalkylene group having 3 to 4 carbon atoms and the oxyethylene group may be added in a block or random manner; and R¹ and R² are each a hydrocarbon group having 1 to 4 carbon atoms or a hydrogen atom, and may be the same or different, and the ratio of the number of hydrogen atoms to the number of hydrocarbon groups in R¹ and R² is 0.15 or less on average.

In the alkylene oxide derivative of formula (I), AO is an oxyalkylene group having 3 to 4 carbon atoms, and specific examples thereof include an oxypropylene group, oxybutylene group, oxyisobutylene group, oxytrimethylene group, and oxytetramethylene group. Preferred examples include an oxypropylene group and oxybutylene group. EO is an oxyethylene group. m is the average number of added moles of oxyethylene groups, and is 1 ≤ m ≤ 70, preferably 2 ≤ m ≤ 50. n is the average number of added moles of oxyalkylene groups having 3 to 4 carbon atoms, and is 1 ≤ n ≤ 70, preferably 5 ≤ n ≤ 55.

The ratio of the oxyethylene group to the total of the oxyalkylene group having 3 to 4 carbon atoms and the oxyethylene group is preferably 20 to 80% by mass. The order of addition of ethylene oxide and alkylene oxide having 3 to 4 carbon atoms is not particularly specified. Further, the oxyethylene group and the oxyalkylene group having 3 to 4 carbon atoms may be added in block form or in random form. They are preferably added in a random manner.

R¹ and R² are each a hydrocarbon group having 1 to 4 carbon atoms or a hydrogen atom, and examples of the hydrocarbon group include alkyl groups such as a methyl group, ethyl group, n-propyl group, isopropyl group, n-butyl group, sec-butyl group, and tert-butyl group. A methyl group or an ethyl group is preferable. Hydrocarbon groups having 5 or more carbon atoms tend to reduce the effects of the present invention. R¹ and R² may be the same or different.

R¹ and R² each may consist of one type, a mixture of a hydrogen atom and a hydrocarbon group having 1 to 4 carbon atoms, or a mixture of hydrocarbon groups having 1 to 4 carbon atoms. However, among the hydrocarbon groups of R¹ and R², the abundance ratio of hydrocarbon groups to hydrogen atoms is such that the ratio Y/X of the number of hydrogen atoms (Y) to the number of hydrocarbon groups (X) is 0.15 or less on average, preferably 0.06 or less on average.

The alkylene oxide derivative of formula (I) can be produced by a known method. For example, it can be obtained by the addition polymerization of ethylene oxide and an alkylene oxide having 3 to 4 carbon atoms to a compound having a hydroxyl group, and then subjecting an alkyl halide to an ether reaction in the presence of an alkali catalyst.

Specific examples of the alkylene oxide derivative of formula (I) include POE (9) POP (2) dimethyl ether, POE (14) POP (7) dimethyl ether, POE (10) POP (10) dimethyl ether, POE (6) POP (14) dimethyl ether, POE (15) POP (5) dimethyl ether, POE (25) POP (25) dimethyl ether, POE (7) POP (12) dimethyl ether, POE (22) POP (40) dimethyl ether, POE (35) POP (40) dimethyl ether, POE (50) POP (40) dimethyl ether, POE (55) POP (30) dimethyl ether, POE (30) POP (34) dimethyl ether, POE (25) POP (30) dimethyl ether, POE (27) POP (14) dimethyl ether, POE (55) POP (28) dimethyl ether, POE (36) POP (41) dimethyl ether, POE (7) POP (12) dimethyl ether, POE (17) POP (4) dimethyl ether, POE (9) POB (2) dimethyl ether, POE (14) POB (7) dimethyl ether, POE (10) POP (10) diethyl ether, POE (10) POP (10) dipropyl ether, and POE (10) POP (10) dibutyl ether. In the present invention, polyoxyethylene (POE) / polyoxypropylene (POP) dimethyl ether is preferably used, but the alkylene oxide derivative is not limited thereto.

Note that the above POE, POP, and POB are abbreviations for polyoxyethylene, polyoxypropylene, and polyoxybutylene, respectively.

One or more alkylene oxide derivatives can be arbitrarily selected and used in the present invention, but the inclusion of a polyoxyethylene/polyoxypropylene dimethyl ether, which is an alkylene oxide derivative of formula (I), and among them, a random polyoxyethylene (14) polyoxypropylene (7) dimethyl ether, is particularly preferable.

The preferred content of the alkylene oxide derivative in the external skin composition of the present invention is, relative to the total of the external skin composition, 0.1% by weight or more, preferably 0.5% by weight or more, and more preferably 1% by weight or more, and is 20% by weight or less, preferably 10% by weight or less, and more preferably 5% by weight or less, and relative to the total, is 0.01 to 99.99% by weight, 0.1 to 99.9% by weight, 0.1 to 20% by weight, 0.1 to 10% by weight, 0.1 to 5% by weight, 0.5 to 20% by weight, 0.5 to 10% by weight, 0.5 to 5% by weight, 1 to 20% by weight, 1 to 10% by weight, or 1 to 5% by weight.

### (Other Components)

The external skin composition of the present invention can contain the following components.

### (1) UV Absorber

UV absorbers absorb incident ultraviolet and are thus considered to indirectly inhibit the function of a UV wavelength conversion substance. However, surprisingly, the external skin composition of the present invention can contain a UV absorber and the function of a UV wavelength conversion substance can be exhibited.

The external skin composition of the present invention contains a UV absorber. The phrase "UV absorber" refers to a substance which absorbs ultraviolet and converts it to the energy of heat or infrared and then emit it. The UV absorber that can be used in the present invention is not particularly limited as long as the function of the UV wavelength conversion substance is not directly impaired. Examples of the UV absorber include salicylic acid-based UV absorbers such as homomenthyl salicylate, ethylhexyl salicylate (octyl salicylate), homosalate, and triethanolamine salicylate;
cinnamic acid-based UV absorbers such as 2-ethylhexyl para-methoxycinnamate, glyceryl diparamethoxycinnamate mono-2-ethylhexanoate, methyl 2,5-diisopropylcinnamate, 2,4,6-tris[4-(2-ethylhexyloxycarbonyl)anilino]-1,3,5-triazine, (hereinafter also referred to as "ethylhexyl triazone"), isopentyl trimethoxycinnamate trisiloxane, an isopropyl para-methoxycinnamate-diisopropylcinnamate ester mixture, and a diethanolamine p-methoxyhydrocinnamate salt;
benzoylmethane UV absorbers such as 2-phenyl-benzimidazole-5-sulfuric acid, 4-isopropyldibenzoylmethane, and 4-tert-butyl-4' -methoxydibenzoylmethane;
octocrylene, Polysilicone-15, 2-ethylhexyl dimethoxybenzylidene dioxoimidazolidine propionate, 1-(3,4-dimethoxyphenyl)-4,4-dimethyl-1,3-pentanedione, cinoxate, methyl-O-aminobenzoate, 3-(4-methylbenzylidene)camphor, octyltriazone, hexyl diethylaminohydroxybenzoyl benzoate, bis(ethylhexyloxyphenol)methoxyphenyl triazine, and methylene bis-benzotriazolyl tetramethylbutylphenol. One or more selected therefrom can be contained in the external skin composition.

The total content of the UV absorber that can be contained in the external skin composition of the present invention is 0.5% by weight or more, preferably 1% by weight or more, more preferably 5% by weight or more, even more preferably 8% by weight or more, or most preferably 10% by weight or more, relative to the total of the external skin composition to function as a sunscreen and 40% by weight or less, preferably 30% by weight or less, more preferably 25% by weight or less, even more preferably 20% by weight or less, relative to the total of the external skin composition to avoid excessive absorption of ultraviolet contained in incident light. The total content of the UV absorber that can be contained in the external skin composition of the present invention is 0.5 to 40% by weight, 1 to 40% by weight, 1 to 30% by weight, 1 to 25% by weight, 1 to 20% by weight, 5 to 40% by weight, 5 to 30% by weight, 5 to 25% by weight, 5 to 20% by weight, 8 to 30% by weight, 8 to 25% by weight, or 8 to 20% by weight.

### (2) UV Scattering Agent

UV scattering agents scatter incident ultraviolet and are thus considered to indirectly inhibit the function of a UV wavelength conversion substance. However, surprisingly, the external skin composition of the present invention can contain a UV scattering agent and the function of a UV wavelength conversion substance can be exhibited.

The external skin composition of the present invention may contain a UV scattering agent. The term "UV scattering agent" refers to a substance which can reflect/scatter ultraviolet and protect skin from ultraviolet. Examples of the material of the UV scattering agent that can be used in the present invention include titanium dioxide, zinc oxide other than component (A), iron oxide, zirconium oxide, and aluminum oxide. The UV scattering agent may be fine particles or a composite thereof. The UV scattering agent preferably contains one or more selected from titanium dioxide and zinc oxide other than component (A).

The titanium dioxide and zinc oxide used as the UV scattering agents may be titanium dioxide and zinc oxide used commonly in cosmetics. Preferably, those having superior dispersibility, such as those subjected to a surface treatment by a publicly known method, as needed, specifically those subjected to a hydrophobic treatment, can be contained in the external skin composition.

Examples of the surface treatment include a silicone treatment with methylhydrogen polysiloxane or methylpolysiloxane; a fluorine treatment with perfluoroalkyl phosphoric acid ester or perfluoroalcohol; an amino acid treatment with N-acylglutamic acid; an alkylalkoxysilane treatment with octyltriethoxysilane or octyltrimethoxysilane; stearic acid/Al hydroxide treatment; and further, a lecithin treatment; a metal soap treatment; a fatty acid treatment; and an alkyl phosphate treatment. Thereamong, zinc oxide surface-treated with silicone is preferably used.

The silicone used for surface treatment is not particularly limited. Examples thereof include methylpolysiloxane, methylphenylpolysiloxane, methylhydrogen polysiloxane, methylcyclopolysiloxane, octamethylcyclotetrasiloxane, decamethylcyclopentasiloxane, dodecamethylcyclohexasiloxane, octamethyltrisiloxane, tetradecamethylhexasiloxane, dimethylsiloxane-methyl(polyoxyethylene)siloxane-methyl(polyoxypropylene)siloxane copolymer, dimethylsiloxane-methyl(polyoxyethylene)siloxane copolymer, dimethylsiloxane-methyl(polyoxypropylene)siloxane copolymer, dimethylsiloxane-methylcetyloxysiloxane copolymer, dimethylsiloxane-methylstearoxysiloxane copolymer, and various other silicones. The silicone is preferably methylhydrogen polysiloxane or methylpolysiloxane.

Among the UV scattering agents that can be included in the external skin composition of the present invention, octyltriethoxysilane-treated zinc oxide can facilitate redispersion of precipitated and aggregated metal compound particles having a large specific gravity.

The total content of the UV scattering agent that can be contained in the external skin composition of the present invention is 0.1% by weight or more, preferably 0.5% by weight or more, more preferably 1% by weight or more, even more preferably 5% by weight or more, or most preferably 10% by weight or more, relative to the total of the external skin composition to function as a sunscreen and 40% by weight or less, preferably 30% by weight or less, more preferably 25% by weight or less, or even more preferably 20% by weight or less, relative to the total of the external skin composition to avoid excessive scattering of ultraviolet contained in incident light. The total content of the UV scattering agent that can be contained in the external skin composition of the present invention is 0.1 to 40% by weight, 0.1 to 30% by weight, 0.1 to 25% by weight, 0.1 to 20% by weight, 0.5 to 40% by weight, 0.5 to 30% by weight, 0.5 to 25% by weight, 0.5 to 20% by weight, 1 to 40% by weight, 1 to 30% by weight, 1 to 25% by weight, 1 to 20% by weight, 5 to 40% by weight, 5 to 30% by weight, 5 to 25% by weight, 5 to 20% by weight, 10 to 40% by weight, 10 to 30% by weight, 10 to 25% by weight, or 10 to 20% by weight.

### (3) Powder

The external skin composition of the present invention may contain a powder. "Powder" refers to a solid substance that has been broken down into very fine pieces. The powder contained in the external skin composition of the present invention is not particularly limited as long as it is commonly used in cosmetics, and examples thereof include polymethyl methacrylate, crosslinked silicone/reticular silicone block copolymer, silica, hydrophobized talc, corn starch, and hydrophobized polyurethane. All or a part of the powder is dispersed in the oil phase of the external skin composition of the invention.

The amount of powder that can be included in the external skin composition of the present invention is 0.01% by weight or more, preferably 0.05% by weight or more, more preferably 0.1% by weight or more, and further preferably 0.5% by weight or more, and is 20% by weight or less, preferably 15% by weight or less, more preferably 10% by weight or less, and further preferably 5% by weight or less relative to the total of the external skin composition, and is 0.01 to 99.99% by weight, 0.1 to 99.9% by weight, 0.1 to 20% by weight, 0.1 to 15% by weight, 0.1 to 10% by weight, 0.5 to 20% by weight %, 0.5 to 10% by weight, or 1 to 10% by weight relative to the total of the external skin composition.

### (4) Dispersant

The external skin composition of the present invention may contain a dispersant. "Dispersant" refers to a substance that can adsorb on the surface of particles dispersed in the water phase or oil phase and uniformly disperse the particles in a water-based or oil-based medium. The dispersant which can be used in the present invention is not particularly limited as long as the function of the UV wavelength conversion substance is not impaired thereby. Oil-based dispersants are preferable, and examples of oil-based dispersants include nonionic surfactants, cationic surfactants, anionic surfactants, silicone-based surfactants, and fatty acids. In the present invention, in particular, the use of a nonionic surfactant, and/or a silicone-based surfactant, and/or a fatty acid which are conventionally used in cosmetics and pharmaceuticals is preferable.

As preferred dispersants which can be contained in the external skin composition of the present invention, PEG-10 dimethicone, bis-butyldimethicone polyglyceryl-3, PEG-polydimethylpolysiloxane ethyldimethicone, lauryl PEG-polydimethylpolysiloxane ethyl dimethicone, cetyl PEG/PPG-10/dimethicone, isostearic acid, diisostearic acid polyglyceryl-2, carboxydecyl trisiloxane, PEG-12 dimethicone, or polyoxyethylene sorbitan monostearate, as well as combinations of two or more of these can be contained.

As preferred examples of dispersants which can be contained in the external skin composition of the present invention, since they enhance the function of the UV wavelength conversion substance, PEG-10 dimethicone, bis-butyldimethicone polyglyceryl-3, PEG-9 polydimethylpolysiloxyethyl dimethicone, lauryl PEG-9 polydimethylpolysiloxyethyl dimethicone, cetyl PEG/PPG-10/1 dimethicone, isostearic acid, or carboxydecyl trisiloxane, as well as combinations of two or more of these can be contained.

The content of the dispersant in the external skin composition of the present invention is not particularly limited as long as the wavelength conversion effect of the present invention is not impaired thereby, and can be appropriately determined in accordance with the type of the UV wavelength conversion substance or the application of the external skin composition containing the UV wavelength conversion substance. For example, the content may be arbitrary in the range of 0.01 to 10% by weight, or 0.1 to 99.9% by weight.

The content of the preferred dispersant in the external skin composition of the present invention is, relative to the total of the external skin composition, 0.1% by weight or more, preferably 0.3% by mass or more, and more preferably 0.5% by weight or more, and is 20% by weight or less, preferably 10% by weight or less, and more preferably 5% by weight or less, and further preferably 2% by weight or less, and is, relative to the total, 0.01 to 99.99% by weight, 0.1 to 99.9% by weight, 0.1 to 20% by weight, 0.1 to 10% by weight, 0.1 to 5% by weight, 0.1 to 3% by weight, 0.1 to 2% by weight, 0.1 to 1% by weight, 0.5 to 10% by weight, 0.5 to 5% by weight, or 0.5 to 2% by weight.

As one aspect of the present invention, the dispersant in the external skin composition contains bisbutyl dimethicone polyglyceryl-3 (KF-6109 manufactured by Shin-Etsu Chemical Co., Ltd., etc.), and the content of dispersant contained in the external skin composition is, relative to the total of the external skin composition, 0.1% by weight or more, preferably 0.3% by mass or more, and more preferably 0.5% by weight or more, and is 20% by weight or less, preferably 10% by weight or less, more preferably 5% by weight or less, and further preferably 2% by weight or less, and is 0.01 to 99.99% by weight, 0.1 to 99.9% by weight, 0.1 to 20% by weight, 0.1 to 10% by weight, 0.1 to 5% by weight, 0.1 to 3% by weight, 0.1 to 2% by weight, 0.1 to 1% by weight, 0.5 to 10% by weight, 0.5 to 5% by weight, or 0.5 to 2% by weight relative to the total of the external skin composition.

### (5) Oil Content

The external skin composition of the present invention contains an oil content. The phrase "oil content" refers to a hydrophobic substance that phase-separates from water, which is a component of the external skin composition of the present invention. The oil content that can be used in the present invention is not particularly limited and contains one or more of, for example, hydrocarbon oils, ester oils, silicone oils, liquid oils, solid fats, and higher alcohols.

Examples of the hydrocarbon oils include liquid paraffin, tetraisobutane, hydrogenated polydecene, olefin oligomer, isododecane, isohexadecane, squalane, and hydrogenated polyisobutene.

Examples of the ester oils include (C₁₂₋₁₅) alkyl benzoate, diisopropyl sebacate, octyl palmitate, cetyl isooctanoate (cetyl 2-ethylhexanoate), triethylhexanoin, neopentyl glycol dicaprate, triisostearin, diisostearyl malate, PPG-3 dipivalate, di-2-ethylhexyl succinate, 2-ethylhexyl 2-ethylhexanoate, polyglyceryl-6 octacaprylate, and glyceryl tri(caprylate/caprate).

Examples of the silicone oils include caprylylmethicone, dimethicone, amino-modified polysiloxane, polyether-modified polysiloxane, alkyl-modified polysiloxane, and fluorine-modified polysiloxane.

Examples of the liquid oils include avocado oil, camellia oil, macadamia nut oil, mink oil, olive oil, castor oil, jojoba oil, triglycerol, glycerol trioctanoate, and isostearic acid.

Examples of the solid fats include coconut oil, hardened coconut oil, palm oil, beef tallow, mutton tallow, Japan wax, and hardened castor oil.

Examples of the higher alcohols include isostearyl alcohol, oleyl alcohol, and butylene glycol-propylene glycol copolymer (e.g., PBG/PPG-9/1 copolymer).

The total content of the oil content that can be contained in the external skin composition of the present invention is 5% by weight or more, preferably 10% by weight or more, and more preferably 15% by weight or more, relative to the total of the external skin composition.

### (6) Oil Phase Thickener

The external skin composition of the present invention may contain an oil phase thickener. An oil phase thickener is an additive that can increase the viscosity of the oil phase liquid by dissolving in the oil phase of the external skin composition. Examples of oil phase thickeners include sugar fatty acid esters, solid oils, metal soaps, 12-hydroxystearic acid, and inorganic polymers such as bentonite and hectorite (e.g., dimethyl distearyl ammonium hectorite). As a sugar fatty acid ester, esters of fatty acids having 10 to 22 carbon atoms, etc., and dextrin, sucrose (e.g., sucrose triacetate tetrastearate), and inulin, and specific examples include dextrin palmitate, dextrin myristate, dextrin (palmitate/ethylhexanoate), dextrin stearate, dextrin behenate, dextrin laurate, coconut oil fatty acid dextrin, sucrose palmitate, sucrose stearate, and inulin stearate. Among these, sucrose triacetate tetrastearate, dimethyl distearyl ammonium hectorite, dextrin palmitate, dextrin myristate, dextrin (palmitate/ethylhexanoate), and inulin stearate are particularly preferable in terms of usability and stability. One or more types selected from these can be included in the external skin composition.

### (7) Others

Various components can be appropriately blended in the external skin composition of the present invention as long as the effect of the present invention is not impaired. Various components are additives that can be normally blended in cosmetics, such as powders ((spherical) silica, polymethyl methacrylate, crosslinked silicone/reticular silicone block copolymer, talc (may be treated with dimethicone), corn starch, polyurethane, etc.), chelating agents (sodium edetate hydrate, etc.), fragrances, moisturizing agents other than (B) (glycerin, dipropylene glycol, PEG-6, etc.), preservatives, anionic surfactants, cationic surfactants, amphoteric surfactants, nonionic surfactants, humectants, water-soluble polymers, film-forming agents such as siliconized polysaccharides and (acrylamide/DMAPA acrylate/methoxy PEG methacrylate) copolymers, metal ion sequestering agents, lower alcohols, polyhydric alcohols, various extracts, sugars, amino acids, organic amines, polymer emulsions, pH adjusters, skin nutrients, vitamins, pharmaceuticals, quasi-drugs, water-soluble drugs applicable to cosmetics (tranexamic acid, niacinamide, potassium 4-methoxysalicylate, dipotassium glycyrrhizinate, etc.), antioxidants, buffering agents, antioxidant aids, propellants, pigments, dyes, colors, water, acid components, and alkaline components. These optional components can be appropriately blended in an oil phase or a water phase. Further, another cell activating agent may be contained or co-used to enhance the effect of the present invention.

The external skin composition of the present invention can be produced by a conventional production method.

Specifically, the external skin composition in this embodiment is obtained by the following procedure. Component (B) and water are mixed to prepare a water phase, and component (A) or (A') is mixed when dispersed in the water phase. An oil phase is prepared by mixing an oil component such as oil, and (A) or (A') is mixed when dispersed in the oil phase. An external skin composition is obtained by adding the oil phase and other components to the water phase and stirring.

The external skin composition of the present invention includes sunscreen cosmetics such as sunscreen cream. Further, the dosage form can be, for example, a milky lotion or cream. The external skin composition of the present invention provides a refreshing feel compared to a water-in-oil type, and has a unique freshness that feels as if it melts and collapses when applied to the skin.

The external skin composition of the present invention can be used by application, preferably coating, thereof to skin, in particular, any of skin excluding hair, preferably face, body, and limbs. For example, by the application, preferably coating, of the external skin composition of the present embodiment, not only can skin be protected from ultraviolet to suppress adverse effects on the skin, the skin can also be imparted with natural and preferable appearance via activation of skin cells.

### UV Wavelength Conversion Function Promoter

As one aspect of the present invention, there is provided an alkylene oxide derivative as a UV wavelength conversion function promoter. In the present invention, "UV wavelength conversion function promoter" means a compound which can enhance the UV wavelength conversion function of a UV wavelength conversion substance in a composition containing the UV wavelength conversion substance, though the compound itself does not have UV wavelength conversion activity.

It can be confirmed that the alkylene oxide derivative is a UV wavelength conversion function promoter by adding the alkylene oxide derivative to a composition containing the UV wavelength conversion substance and observing that the fluorescence intensity increases when the composition is irradiated with ultraviolet. To measure the fluorescence intensity, for example, the fluorescence intensity when a plate containing the external skin composition is irradiated with ultraviolet may be measured using a spectrofluorometer, or fluorescence intensity can be measured using a spectrofluorometer by irradiating with ultraviolet a coating film of the composition on the surface of a substrate. The substrate may be a resin substrate composed of a polymethyl methacrylate (PMMA), nylon, or acrylic plate or an inorganic plate composed of glass or quartz. For example, a PMMA plate having V-shaped grooves on its surface (also refer to as "S plate": Japanese Registered Patent Publication No. 4453995) can be used. As the fluorescence intensity, a fluorescence value at a specific single wavelength or an integrated value in a specific wavelength region may be used.

As the alkylene oxide derivative as the UV wavelength conversion function promoter of the present invention, it is particularly preferable to use an alkylene oxide derivative represented by the following formula (I):

R¹O-[(EO)ₘ(AO)ₙ]-R² (I)

where AO is an oxyalkylene group having 3 to 4 carbon atoms, EO is an oxyethylene group, m and n are the average numbers of added moles of the oxyethylene group and the oxyalkylene group, respectively, and are 1 ≤ m ≤ 70 and 1 ≤ n ≤ 70; the ratio of oxyethylene groups to the total of oxyalkylene groups having 3 to 4 carbon atoms and oxyethylene groups is 20 to 80% by mass (0.2 ≤ m / (m + n) ≤ 0.8); the oxyalkylene group having 3 to 4 carbon atoms and the oxyethylene group may be added in a block or random manner; and R¹ and R² are each a hydrocarbon group having 1 to 4 carbon atoms or a hydrogen atom, and may be the same or different, and the ratio of the number of hydrogen atoms to the number of hydrocarbon groups in R¹ and R² is 0.15 or less on average.

In the alkylene oxide derivative of formula (I), AO is an oxyalkylene group having 3 to 4 carbon atoms, and specific examples thereof include an oxypropylene group, oxybutylene group, oxyisobutylene group, oxytrimethylene group, and oxytetramethylene group. Preferred examples include oxypropylene group and oxybutylene group. EO is an oxyethylene group. m is the average number of added moles of oxyethylene groups, and is 1 ≤ m ≤ 70, and preferably 2 ≤ m ≤ 50. n is the average number of added moles of oxyalkylene groups having 3 to 4 carbon atoms, and is 1 ≤ n ≤ 70, and preferably 5 ≤ n ≤ 55.

Further, the ratio of the oxyethylene group to the total of the oxyalkylene group having 3 to 4 carbon atoms and the oxyethylene group is preferably 20 to 80% by mass. The order of addition of ethylene oxide and alkylene oxide having 3 to 4 carbon atoms is not particularly specified. Further, the oxyethylene group and the oxyalkylene group having 3 to 4 carbon atoms may be added in block form or in random form. It is preferable that they be added in a random manner.

R¹ and R² are each a hydrocarbon group having 1 to 4 carbon atoms or a hydrogen atom, and examples of the hydrocarbon groups include alkyl groups such as a methyl group, ethyl group, n-propyl group, isopropyl group, n-butyl group, sec-butyl group, tert-butyl group. A methyl group or an ethyl group is preferable. Hydrocarbon groups having 5 or more carbon atoms tend to reduce the effects of the present invention. R¹ and R² may be the same or different.

Only one type of each of R¹ and R² may be used, or a hydrocarbon group having 1 to 4 carbon atoms and a hydrogen atom may be mixed, or hydrocarbon groups having 1 to 4 carbon atoms may be mixed. However, among the hydrocarbon groups of R¹ and R², the abundance ratio of hydrocarbon groups to hydrogen atoms is such that the ratio Y/X of the number of hydrogen atoms (Y) to the number of hydrocarbon groups (X) is 0.15 or less on average, preferably 0.06 or less on average.

The alkylene oxide derivative of formula (I) can be produced by a known method. For example, it can be obtained by the addition polymerization of ethylene oxide and an alkylene oxide having 3 to 4 carbon atoms to a compound having a hydroxyl group, and then subjecting the alkyl halide to an ether reaction in the presence of an alkali catalyst.

Specific examples of the alkylene oxide derivative of formula (I) include POE (9) POP (2) dimethyl ether, POE (14) POP (7) dimethyl ether, POE (10) POP (10) dimethyl ether, POE (6) POP (14) dimethyl ether, POE (15) POP (5) dimethyl ether, POE (25) POP (25) dimethyl ether, POE (7) POP (12) dimethyl ether, POE (22) POP (40) dimethyl ether, POE (35) POP (40) dimethyl ether, POE (50) POP (40) dimethyl ether, POE (55) POP (30) dimethyl ether, POE (30) POP (34) dimethyl ether, POE (25) POP (30) dimethyl ether, POE (27) POP (14) dimethyl ether, POE (55) POP (28) dimethyl ether, POE (36) POP (41) dimethyl ether, POE (7) POP (12) dimethyl ether, POE (17) POP (4) dimethyl ether, POE (9) POB (2) dimethyl ether, POE (14) POB (7) dimethyl ether, POE (10) POP (10) diethyl ether, POE (10) POP (10) dipropyl ether, and POE (10) POP (10) dibutyl ether. In the present invention, polyoxyethylene (POE) / polyoxypropylene (POP) dimethyl ether is preferably used, but the alkylene oxide derivative is not limited thereto.

Note that the above POE, POP, and POB are abbreviations for polyoxyethylene, polyoxypropylene, and polyoxybutylene, respectively.

One or more alkylene oxide derivatives can be arbitrarily selected and used in the present invention, but the inclusion of a polyoxyethylene/polyoxypropylene dimethyl ether, which is an alkylene oxide derivative of formula (I), and among them, a random polyoxyethylene (14) polyoxypropylene (7) dimethyl ether, is particularly preferable.

The content of the alkylene oxide derivative as the UV wavelength conversion function promoter of the present invention in the composition containing the UV wavelength conversion substance is, relative to the total composition, 0.1% by weight or more, preferably 0.5% by mass or more, and more preferably 1% by weight or more, and is 20% by weight or less, preferably 10% by weight or less, and more preferably 5% by weight or less, and relative to the total, is 0.01 to 99.99% by weight, 0.1 to 99.9% by weight, 0.1 to 20% by weight, 0.1 to 10% by weight, 0.1 to 5% by weight, 0.5 to 20% by weight, 0.5 to 10% by weight, 0.5 to 5% by weight, 1 to 20% by weight, 1 to 10% by weight, or 1 to 5% by weight.

Examples of the UV wavelength conversion substance in which the alkylene oxide derivative of the present invention acts as a UV wavelength conversion function promoter include the following components: phycobiliproteins such as phycocyanin (allophycocyanin, C-phycocyanin, R-phycocyanin), phycoerythrocyanin, phycoerythrin (B-phycoerythrin, b-phycoerythrin, C-phycoerythrin, R-phycoerythrin); natural or synthetic components such as vitamin A, β-carotene, vitamin K, vitamin B1, vitamin B2, vitamin B6, vitamin B12, folic acid, niacin, salicylic acid, lycopene, capsicum extract, capsicum color, paprika color, gardenia color, safflower color, turmeric color, cochineal color, perilla color, red cabbage color, flavonoid, carotenoid, quinoid, porphyrins, anthocyanins, and polyphenols; colors such as Red No. 401, Red No. 227, Red No. 504, Red No. 218, Orange No. 205P, Yellow No. 4, Yellow No. 5, Green No. 201, pyranine conc., Blue No. 1, 2,4-diaminophenoxyethanol hydrochloride, Alizuline Purple SS, Purple No. 401, Black No. 401, Herindon pink, Yellow No. 401, Benzidine yellow G, Blue No. 404, Red No. 104, and meta-aminophenol; phosphors of an inorganic compound doped to exhibit a fluorescence property, such as the blue phosphor containing an amorphous silica particle, cerium, and phosphorus and/or magnesium described in Japanese Registered Patent Publication No. 6424656, the red phosphor containing a europium-activated compound of a mixed crystal of an alkaline earth metal sulfide and a gallium compound described in Japanese Registered Patent Publication No. 6361416; the zinc oxide phosphor described in WO 2018/004006; the zinc oxide phosphor described in Japanese Unexamined Patent Publication (Kokai) No. 2018-131422; and the inorganic phosphor described in Japanese Unexamined Patent Publication (Kokai) No. 5-117127 (hereinafter, a phosphor derived from zinc oxide is referred to as a "zinc oxide phosphor"). In one embodiment, the inorganic phosphor is one or more phosphors selected from phosphors of zinc oxide represented by ZnO:Zn, Zn_{1+z}, or ZnO₁₋ₓ, doped with a sulfide and/or a sulfate, such as zinc sulfide and zinc sulfate, described in WO 2018/004006; magnesium titanate phosphors of a magnesium titanate such as MgTiO₃ or Mg₂TiO₄ doped with manganese (hereinafter a phosphor derived from magnesium titanate is referred to as a "magnesium titanate phosphor"); and calcium phosphate phosphors of a calcium phosphate such as Ca(H₂PO₄)₂, CaHPO₄, or Ca₃(PO₄)₂ doped with cerium.

As one aspect of the invention, there is provided the use of an alkylene oxide derivative to enhance the functionality of a UV wavelength conversion substance. The alkylene oxide derivative can be added as a component of the composition containing the UV wavelength conversion substance. As the alkylene oxide derivative, the use of an alkylene oxide derivative represented by the following formula (I) is particularly preferable:

R¹O-[(EO)ₘ(AO)ₙ]-R² (I)

where AO is an oxyalkylene group having 3 to 4 carbon atoms, EO is an oxyethylene group, m and n are the average numbers of added moles of the oxyethylene group and the oxyalkylene group, respectively, and are 1 ≤ m ≤ 70 and 1 ≤ n ≤ 70; the ratio of oxyethylene groups to the total of oxyalkylene groups having 3 to 4 carbon atoms and oxyethylene groups is 20 to 80% by mass (0.2 ≤ m / (m + n) ≤ 0.8); the oxyalkylene group having 3 to 4 carbon atoms and the oxyethylene group may be added in a block or random manner; and R¹ and R² are each a hydrocarbon group having 1 to 4 carbon atoms or a hydrogen atom, and may be the same or different, and the ratio of the number of hydrogen atoms to the number of hydrocarbon groups in R¹ and R² is 0.15 or less on average.

Though the reason why the alkylene oxide derivative of the present invention exhibits excellent effects as a UV wavelength conversion function promoter is not clear, presumably, due to its composition that functions as a humectant and has two different properties such as POE (14) and POP (7), the alkylene oxide derivative increases the affinity between the UV wavelength conversion substance and the base, and that the UV wavelength conversion substance is homogeneously dispersed in the base, thereby efficiently converting the wavelength of the irradiated ultraviolet rays.

### EXAMPLES

The present invention will be described in more detail with reference to the following examples. Note that the present invention is not limited thereto.

### Example 1: Effects of POE (14) POP (7) dimethyl ether on UV Wavelength Conversion Substance

External skin compositions (Formulation Examples A and B) having the compositions shown in Table 1 were produced according to a conventional production method. The POE (14) POP (7) dimethyl ether used had oxypropylene groups and oxyethylene groups added in a random manner. Each external skin composition also includes octyltriethoxysilane-treated zinc oxide phosphor as a UV wavelength conversion substance. The obtained external skin compositions were irradiated with ultraviolet having a wavelength of 365 nm, and the integrated fluorescence value of 400 to 600 nm was measured using a spectrofluorometer RF-5300PC (Shimadzu Corporation).

When POE (14) POP (7) dimethyl ether, known as a humectant, was added to Formulation Example A at a concentration of 2%, the integrated fluorescence value due to the zinc oxide phosphor treated with octyltriethoxysilane, which is a UV wavelength conversion substance, was 133%, which was found to be significantly increased (Formulation Example B) (p < 0.05 (n = 5)). When octyltriethoxysilane-treated zinc oxide phosphor was not included, even when 2% of POE (14) POP (7) dimethyl ether was added, the integrated fluorescence value did not increase, and POE (14) POP (7) dimethyl ether itself did not function as a UV wavelength conversion substance.

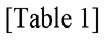

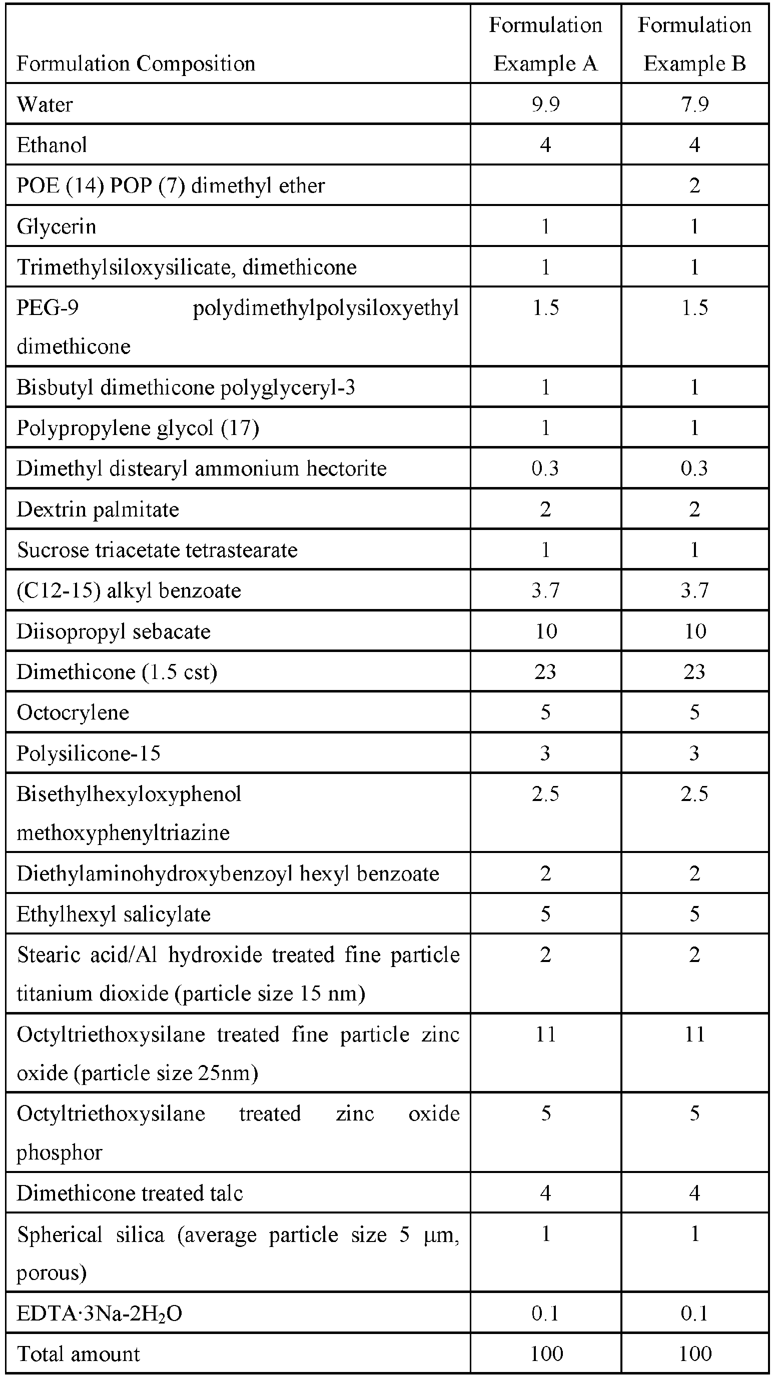

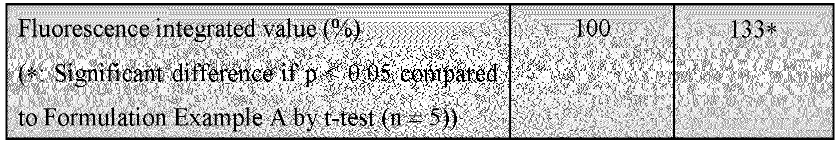

### Example 2: Effects of Humectants on UV Wavelength Conversion Substance (Zinc Oxide Phosphor)

External skin compositions (Formulation Examples C to F) having the compositions shown in Table 2 were produced according to a conventional production method. Formulation Example C did not contain a moisturizer, Formulation Example D contained 1% POE (14) POP (7) dimethyl ether (added in a random manner), Formulation Example E contained 2% glycerin, and Formulation Example F contained 2% PEG-6. Though glycerin and PEG-6 enhanced the ultraviolet wavelength conversion function of the octyltriethoxysilane-treated zinc oxide phosphor, but not significantly (p > 0.05 (n = 5)), and even when POE (14) POP (7) dimethyl ether was lowered to a concentration of 1%, the ultraviolet wavelength conversion function of the octyltriethoxysilane-treated zinc oxide phosphor was significantly enhanced (p < 0.005 (n = 5)).

**Table 2**

| Formulation Composition | Formulation Example C | Formulation Example D | Formulation Example E | Formulation Example F |
|---|---|---|---|---|
| Water | 10.9 | 9.9 | 8.9 | 8.9 |
| Ethanol | 4 | 4 | 4 | 4 |
| POE (14) POP (7) dimethyl ether | | 1 | | |
| Glycerin | | | 2 | |
| PEG-6 | | | | 2 |
| Trimethyl siloxysilicate | 1 | 1 | 1 | 1 |
| PEG-9 polydimethylpolysiloxyethyl dimethicone | 1.5 | 1.5 | 1.5 | 1.5 |
| Bisbutyl dimethicone polyglyceryl-3 | 1 | 1 | 1 | 1 |
| Polypropylene glycol (17) | 1 | 1 | 1 | 1 |
| Dimethyl distearyl ammonium hectorite | 0.3 | 0.3 | 0.3 | 0.3 |
| Dextrin palmitate | 2 | 2 | 2 | 2 |
| Sucrose triacetate tetrastearate | 1 | 1 | 1 | 1 |
| (C₁₂₋₁₅) alkyl benzoate | 3.7 | 3.7 | 3.7 | 3.7 |
| Diisopropyl sebacate | 10 | 10 | 10 | 10 |
| Dimethicone (1.5 cst) | 23 | 23 | 23 | 23 |
| Octocrylene | 5 | 5 | 5 | 5 |
| Polysilicone-15 | 3 | 3 | 3 | 3 |
| Bisethylhexyloxyphenol methoxyphenyltriazine | 2.5 | 2.5 | 2.5 | 2.5 |
| Diethylaminohydroxybenzoyl hexyl benzoate | 2 | 2 | 2 | 2 |
| Ethylhexyl salicylate | 5 | 5 | 5 | 5 |
| Stearic acid/Al hydroxide treated fine particle titanium dioxide (particle size 15 nm) | 2 | 2 | 2 | 2 |
| Octyltriethoxysilane treated fine particle zinc oxide (particle size 25 nm) | 11 | 11 | 11 | 11 |
| Octyltriethoxysilane treated zinc oxide phosphor | 5 | 5 | 5 | 5 |
| Dimethicone treated talc | 4 | 4 | 4 | 4 |
| Spherical silica (average particle size 5 µm, porous) | 1 | 1 | 1 | 1 |
| EDTA·3Na-2H₂O | 0.1 | 0.1 | 0.1 | 0.1 |
| Total amount | 100 | 100 | 100 | 100 |
| Fluorescence integrated value (%) | 100 | 117 | 109 | 106 |
| (***: Significant difference if p < 0.005 compared to Formulation Example C by t-test (n = 5); N.S.: No significant difference compared to Formulation Example C by t-test (p > 0.05, n = 5). ) | | *** | N.S. | N.S. |

### Example 3: Effects of Moisturizer on UV Wavelength Conversion Substance (Zinc Oxide Phosphor + Phycocyanin)

Skin preparations for external use (Formulation Examples G and H) containing phycocyanin (LinaBlue, manufactured by DIC), which is an organic phosphor and has long-wavelength fluorescence, together with a zinc oxide phosphor were produced according to a conventional production method. Formulation Example G contained no humectant, while Formulation Example H contained 2% POE (14) POP (7) dimethyl ether (added in a random manner). The obtained external skin compositions were irradiated with ultraviolet having a wavelength of 350 nm, and the integrated fluorescence value of 400 to 800 nm was measured using a spectrofluorometer RF-5300PC (Shimadzu Corporation). POE (14) POP (7) dimethyl ether enhanced the ultraviolet wavelength conversion function of the octyltriethoxysilane-treated zinc oxide phosphor and LinaBlue (Table 3).

Despite its relatively large molecular weight, POE (14) POP (7) dimethyl ether has the property of being miscible with both water and polar oil without restriction, and it is considered that due to the structure of POE (14) and POP (7) having two different properties, the affinity between the UV wavelength conversion substance and the base was increased, whereby the UV wavelength conversion substance could be homogeneously dispersed in the base to efficiently convert the wavelength of the irradiated ultraviolet.

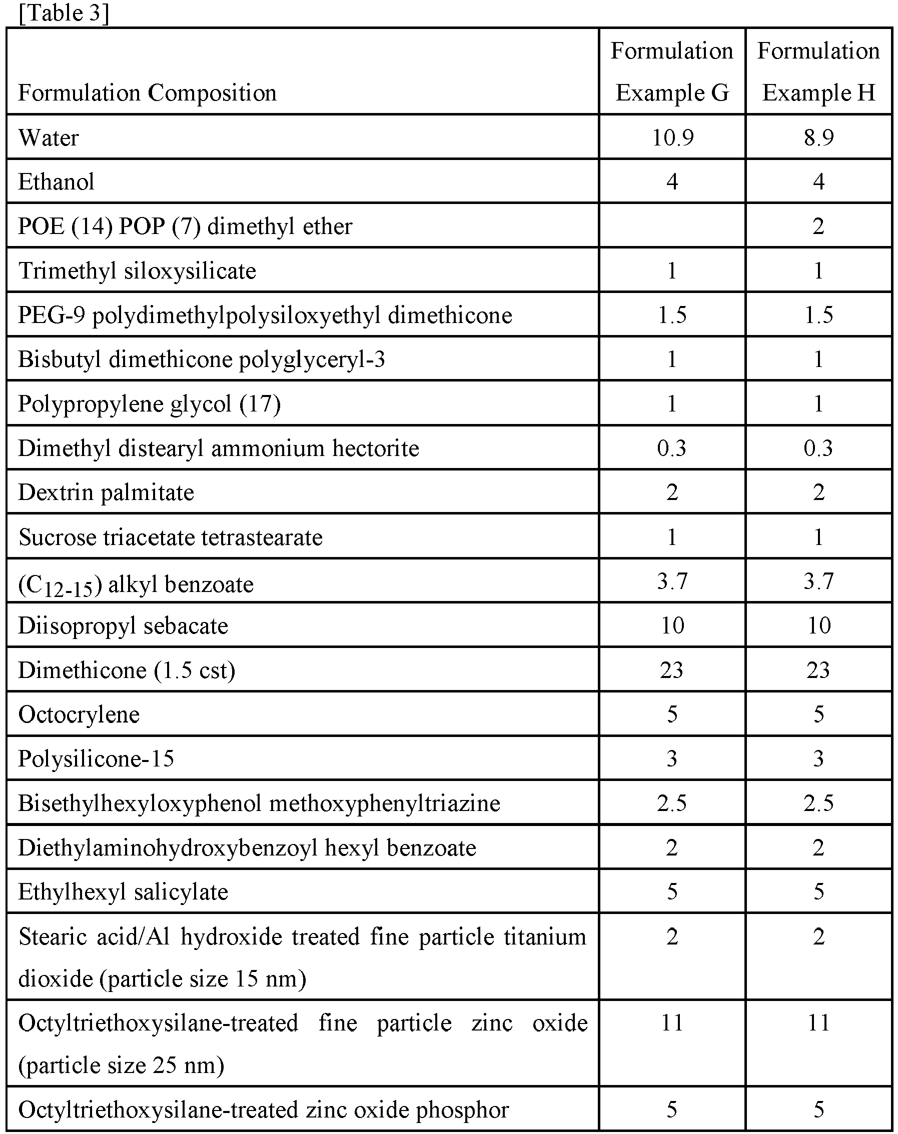

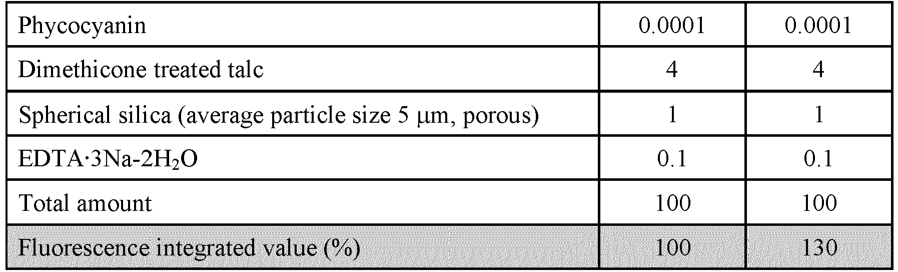

The embodiments of the external skin composition and UV wavelength conversion function promoter of the present invention have been described above. However, the present invention is not limited thereto, and can be modified as appropriate without departing from the spirit of the invention.

## Claims

1. An external skin composition, comprising:
(A) a UV wavelength conversion substance, and
(B) an alkylene oxide derivative represented by formula (I) below:
R¹O-[(EO)ₘ(AO)ₙ]-R² (I)
where
AO is an oxyalkylene group having 3 to 4 carbon atoms,
EO is an oxyethylene group,
the oxyalkylene group having 3 to 4 carbon atoms and the oxyethylene group may be added in a block or random manner,
m and n are average numbers of added moles of the oxyethylene group and the oxyalkylene group, respectively, and 1 ≤ m ≤ 70, 1 ≤ n ≤ 70, and 0.2 ≤ m/(m + n) ≤ 0.8,
R¹ and R² are an alkyl group having 1 to 4 carbon atoms or a hydrogen atom, and may be the same or different,
the ratio of the number of hydrogen atoms in R¹ and R² to the number of alkyl groups in R¹ and R² is 0.15 or less on average.

2. The external skin composition according to claim 1, wherein the UV wavelength conversion substance is one or more selected from a zinc oxide phosphor, a magnesium titanate phosphor, and phycocyanin.

3. The external skin composition according to claim 1 or 2, wherein the UV wavelength conversion substance is a zinc oxide phosphor and phycocyanin.

4. The external skin composition according to any one of claims 1 to 3, wherein the oxyalkylene group and the oxyethylene group of the alkylene oxide derivative are added in a random manner.

5. The external skin composition according to any one of claims 1 to 4, wherein the oxyalkylene group of the alkylene oxide derivative is an oxypropylene group.

6. The external skin composition according to any one of claims 1 to 5, wherein R¹ and R² of the alkylene oxide derivative are both methyl groups.

7. The external skin composition according to any one of claims 1 to 6, comprising 1% by weight or more of the alkylene oxide derivative.

8. The external skin composition according to any one of claims 1 to 7, which is a water-in-oil composition.

9. The external skin composition according to any one of claims 1 to 8, which is for cell activation.

10. A UV wavelength conversion function promoter of a UV wavelength conversion substance, comprising an alkylene oxide derivative represented by the following general formula (I) as an active ingredient:
R¹O-[(EO)ₘ(AO)ₙ]-R² (I)
where
AO is an oxyalkylene group having 3 to 4 carbon atoms,
EO is an oxyethylene group,
the oxyalkylene group having 3 to 4 carbon atoms and the oxyethylene group may be added in a block or random manner,
m and n are average numbers of added moles of the oxyethylene group and the oxyalkylene group, respectively, and 1 ≤ m ≤ 70, 1 ≤ n ≤ 70, and 0.2 ≤ m/(m + n) ≤ 0.8,
R¹ and R² are an alkyl group having 1 to 4 carbon atoms or a hydrogen atom, and may be the same or different,
the ratio of the number of hydrogen atoms in R¹ and R² to the number of alkyl groups in R¹ and R² is 0.15 or less on average.

11. The UV wavelength conversion function promoter according to claim 10, wherein the UV wavelength conversion substance is one or more selected from a zinc oxide phosphor, a magnesium titanate phosphor, and phycocyanin.

12. The UV wavelength conversion function promoter according to claim 10 or 11, wherein the oxyalkylene group and the oxyethylene group of the alkylene oxide derivative are added in a random manner.

13. The UV wavelength conversion function promoter according to any one of claims 10 to 12, wherein the oxyalkylene group of the alkylene oxide derivative is an oxypropylene group.

14. The UV wavelength conversion function promoter according to any one of claims 10 to 13, wherein R¹ and R² of the alkylene oxide derivative are both methyl groups.

15. Use of an alkylene oxide derivative represented by the following formula (I), for promoting a function of a UV wavelength conversion substance:
R¹O-[(EO)ₘ(AO)ₙ]-R² (I)
where
AO is an oxyalkylene group having 3 to 4 carbon atoms,
EO is an oxyethylene group,
the oxyalkylene group having 3 to 4 carbon atoms and the oxyethylene group may be added in a block or random manner
m and n are average numbers of added moles of the oxyethylene group and the oxyalkylene group, respectively, and 1 ≤ m ≤ 70, 1 ≤ n ≤ 70, and 0.2 ≤ m/(m + n) ≤ 0.8,
R¹ and R² are an alkyl group having 1 to 4 carbon atoms or a hydrogen atom, and may be the same or different,
the ratio of the number of hydrogen atoms in R¹ and R² to the number of alkyl groups in R¹ and R² is 0.15 or less on average.
